Europäisches Patentamt

(19) European Patent Office

Office européen des brevets

(11) EP 0 670 490 B1

(12) EUROPÄISCHE PATENTSCHRIFT

(45) Veröffentlichungstag und Bekanntmachung des Hinweises auf die Patenterteilung:
**04.12.2002 Patentblatt 2002/49**

(51) Int Cl.$^7$: **G01N 33/00**, G05D 11/02, G01N 27/00, G01N 1/22, G05D 11/13

(21) Anmeldenummer: **95103134.3**

(22) Anmeldetag: **04.03.1995**

(54) **Verfahren und Vorrichtung zum Messen eines Gasmediums mit einem chemischen Sensor**

Method and apparatus for measuring a gaseous medium with a chemical sensor

Méthode et appareil pour la mesure d'un milieu gazeux avec un détecteur chimique

(84) Benannte Vertragsstaaten:
**AT BE DE FR GB IT NL**

(30) Priorität: **05.03.1994 DE 4407345**

(43) Veröffentlichungstag der Anmeldung:
**06.09.1995 Patentblatt 1995/36**

(73) Patentinhaber: **TESTO GmbH & Co.**
**D-79849 Lenzkirch (DE)**

(72) Erfinder:
 • **Brödel, Axel**
 **D-79853 Lenzkirch (DE)**
 • **Springmann, Thomas**
 **D-79102 Freiburg (DE)**
 • **Münch, Reinhold**
 **D-79100 Freiburg (DE)**
 • **Bader, Armin**
 **D-78199 Bräunlingen (DE)**

(74) Vertreter: **Patentanwälte**
**Westphal, Mussgnug & Partner**
**Waldstrasse 33**
**78048 Villingen-Schwenningen (DE)**

(56) Entgegenhaltungen:
 **EP-A- 0 278 520      DD-A- 285 650**
 **DD-A- 285 651        DD-A- 285 652**
 **DE-A- 3 622 354**

**Beschreibung**

Die Erfindung betrifft ein Verfahren und eine Vorrichtung zum Messen eines Gasmediums mit mindestens einem chemischen Sensor.

Chemische bzw. elektrochemische Sensoren und insbesondere Gassensoren für gasförmige Medien arbeiten außerhalb ihres nominellen, d.h. optimalen Meßbereiches nur unzufriedenstellend. Darüberhinaus können außerhalb dieses nominellen Meßbereichs auch Beschädigungen der jeweiligen Sensoren auftreten, die es gilt zu vermeiden. Andererseits sollen solche chemische Sensoren auch außerhalb ihrer nominellen Meßbereiche für Gaskonzentrationsmessungen eingesetzt werden können.

Es sind bereits eine Vielzahl von Veröffentlichungen bekannt, die sich mit der Problematik beschäftigen, bei Detektionssystemen mit Sensoren einen nominellen Meßbereich einzuhalten. Hierfür wird z. B. eine veränderbare Verdünnung des zu messenden Probenmediums vorgeschlagen. So ist ein PAH-Analysator für Echtzeitmessung von Verbrennungsaerosolen mit der Bezeichnung PAS 1000e der Firma Seefelder Meßtechnik GmbH & Co. Vertriebs-KG, Seefeld, bekannt, der beispielsweise zur Luftreinheitsüberwachung eingesetzt wird. Zur Einhaltung des Meßbereiches des dort verwendeten fotoelektrischen Sensors ist eine Verdünnung des zu messenden Aerosoles vorgesehen. Das Verdünnungsverhältnis wird dabei manuell über einen Drehschalter von Hand eingestellt. Durch die Verdünnung des zu messenden Aerosoles wird eine sichere Messung im linearen Bereich des fotoelektrischen Sensors gewährleistet.

Aus den Patentschriften DD 285 650 A5, DD 285 651 A5 sowie DD 285 652 A5 ist dagegen ein dynamisches Verfahren für die Verdünnung eines Meßmediums zur Einhaltung des Meßbereiches eines Detektionssystemes bekannt. Die in diesen drei Druckschriften beschriebenen Verfahren gehen davon aus, daß eine manuelle Einstellung der Verdünnung zur Einhaltung des Meßbereichs von Detektionssystemen problematisch ist. Es ist deshalb ein Regelkreis für eine prozeßorientierte Nachführung der Verdünnung beschrieben. Der Regelkreis sorgt für eine dynamische Nachregulierung des Verdünnungsverhältnisses durch Auswertung des Detektionssignales des Sensors derart, daß die vom Sensor erfaßbare Mischkonzentration im optimalen und damit nominellen Meßbereich des Sensors gehalten wird. Das eigentliche Meßergebnis wird durch Auswertung des Detektionssignales des Sensores unter Berücksichtigung des Verdünnungsverhältnisses ermittelt. Als Meßmedium ist stets eine Flüssigkeit vorgesehen. Das eingestellte Verdünnungsverhältnis wird jeweils über eine Volumenstrommessung bestimmt. Hierzu dient eine Verdünnungsanordnung, die beispielsweise in neun Verdünnungsschritten, beginnend mit einem Verdünnungsverhältnis von 1 : 256 in dualer Verminderungsreihenfolge bis zu einem Verdünnungsverhältnis von 1 : 1 das zu messende Flüssigkeitsmedium verdünnt.

Problematisch bei dieser Volumenstrommessung ist die Verhältnismäßig komplizierte Apparatur. So müssen einerseits (vgl. DD 285 651 A5) Dosiergefäße mit fest vorgegebenen Volumen vorgesehen werden, welche zudem in einem festen vorgegebenen Verhältnis zueinander stehen müssen. Zur Erreichung definierter Mischungsverhältnisse müssen diese Dosiergefäße rechnergesteuert miteinander verknüpft werden. Dabei sind auch die von den Dosiergefäßen von und bis zu den zugehörigen Ventilen notwendigen Leitungsstücke zu berücksichtigen. Andererseits (vgl. DD 285 652 A5) müssen zur Volumenstrommessung eine Pumpanordnung und Schläuche zum Transport des zu messenden Mediums sowie Mittel zur Änderung des Durchflußquerschnittes der Schläuche vorgesehen werden, um nach dem Hagen-Poiseuilleschen Gesetz durch die Schläuche das jeweilige Flüssigkeitsvolumen bestimmen zu können.

Die der Erfindung zugrundeliegende Aufgabe liegt darin, ein Verfahren und eine Vorrichtung zum Messen eines Gasmediums mit mindestens einem chemischen Sensor anzugeben, dessen nomineller Meßbereich auch bei Konzentrationswerten des Gasmediums außerhalb dieses nominellen Meßbereiches sicher eingehalten wird und in einfacher Weise zu realisieren ist.

Diese Aufgabe wird für das Verfahren durch die Merkmale des Anspruchs 1 und für die Vorrichtung durch die Merkmale des Anspruchs 11 gelöst.

Das erfindungsgemäße Verfahren beruht im wesentlichen darauf, einen Probegasstrom mit einem Verdünnungsgasstrom in einem einstellbaren Verdünnungsverhältnis zu vermischen und das Verdünnungsverhältnis durch eine Stoffkonzentrationsmessung einer vorgegebenen Stoffkomponente zumindest nach dem Mischvorgang zu bestimmen. Das Gasgemisch wird erfindungsgemäß dem chemischen Sensor zugeführt, der einen nominellen Meßbereich zum Erzeugen eines Detektionssignales aufweist. Das Verdünnungsverhältnis wird durch Auswertung des Detektionssignales des chemischen Sensors nachreguliert, so daß die vom chemischen Sensor erfaßbare Gasgemischkonzentration sicher im nominellen Meßbereich des chemischen Sensors gehalten wird. Der eigentliche Meßwert für das Gasmedium wird durch Auswertung des Detektionssignales unter Berücksichtigung des eingestellten Verdünnungsverhältnisses ermittelt.

Mit diesem erfindungsgemäßen Verfahren ist die Zusammensetzung des zu messenden Gasmediums über einen großen Konzentrationsbereich mit hoher Genauigkeit detektierbar und damit vor allem für Detektionssysteme mit elektrochemischen Sensoren beim Einsatz in Industrie und Umweltschutz in weitem Umfang anwendbar.

Erfindungsgemäß kann z. B. über eine Probenahmesonde kontinuierlich ein Probegasstrom entnommen werden. Ein in seiner Zusammensetzung bekannter Verdünnungsstrom wird in einer durch Messung bestimmten definierten Größe in einer Mischanordnung dem Probegasstrom beigemischt, so daß über die Detektion der Zielmeßgröße das Verdünnungsverhältnis dynamisch eingestellt bzw. nachgeregelt werden kann. Durch wiederholte Messung der Stoffkomponente im Gasgemischstrom wird das Verdünnungsverhältnis errechnet und zur Meßwertbestimmung sowie Nachregelung benutzt. Hierfür eignet sich insbesondere eine elektronische Steuerung mittels Mikroprozessor. Das erfindungsgemäße Verfahren ist zum Messen von Gasmedien mit komplexer Zusammensetzung und damit insbesondere zur Prozeßgas- und Emissionsmessung einsetzbar.

Das erfindungsgemäße Verfahren ist auch dort einsetzbar, wo sprunghafte Änderungen der Stoffkonzentration des zu messenden Gasmediums auftreten oder der Ausgangswert der Stoffkonzentration im Probegasstrom in der Größenordnung unbekannt ist, da in einer vorteilhaften Weiterbildung der Erfindung zunächst mit einem so hohen Verdünnungsverhältnis gearbeitet wird, daß der chemische Sensor sicher in seinem nominellen Meßbereich arbeitet. Eine Überbelastung bzw. Beschädigung des chemischen Sensores und eine damit verbundene Einschränkung der Qualität des Meßergebnisses wird somit erfindungsgemäß wirksam vermieden.

Gemäß einer Weiterbildung der Erfindung liegt die vorgegebene Stoffkomponente, die beispielsweise Sauerstoff sein kann, im Verdünnungsgasstrom in bekannter und konstanter Größe vor. Die Stoffkonzentrationsmessung dieser Stoffkomponente erfolgt dann sowohl im Probegasstrom als auch im Gasgemischstrom. Es sind also lediglich zwei Sensoren, z. B. Sauerstoffsensoren, zum Erfassen der Sauerstoffkomponente im Probegasstrom und im Gasgemischstrom erforderlich.

Ist die vorgegebene Stoffkomponente gemäß einer weiteren Ausbildung der Erfindung nicht im Probegasstrom vorhanden und liegt die vorgegebene Stoffkomponente im Verdünnungsgasstrom in bekannter und konstanter Größe vor, so kann die erfindungsgemäße Stoffkonzentrationsmessung durch Bestimmung der Stoffkomponente ausschließlich im Gasgemischstrom erfolgen. Hierdurch ergibt sich ein besonders einfacher Aufbau einer Vorrichtung zur Durchführung des Verfahrens, da neben dem chemischen Sensor lediglich ein einziger weiterer Sensor vorgesehen werden muß.

Als Verdünnungsgasstrom hat sich der Einsatz eines Luftstromes als vorteilhaft erwiesen, da keine gesonderten Behältnisse für ein Verdünnungsmedium zur Verfügung gestellt werden müssen. Da Luft ohnehin überall vorhanden ist, reicht es aus, die Luft über ein möglicherweise noch notwendiges Luftfilter zu führen und diesen Luftstrom als Verdünnungsgasstrom mit dem Probegasstrom zu vermischen. Ist als Stoffkomponente beispielsweise Sauerstoff im Probegasstrom nicht vorhanden, so kann durch Detektion des Sauerstoffgehaltes im Gasgemisch in einfacher Weise das Verdünnungsverhältnis von Probegasstrom und Verdünnungsgasstrom ermittelt werden. Ist die Stoffkomponente, hier Sauerstoff, im Probegasstrom nicht vorhanden, so kann selbstverständlich probengasseitig auf die Detektion dieser Stoffkomponente verzichtet werden.

Eine erfindungsgemäße Weiterbildung sieht vor, daß mehrere chemische Sensoren zur Detektion mehrerer Stoffgrößen des Gasmediums vorgesehen sind und eine Regelung des Verdünnungsverhältnisses nach Maßgabe eines Detektionssignales desjenigen chemischen Sensors erfolgt, von dessen nominellen Meßbereich voraussichtlich zu Beginn des Verfahrens am weitesten abgewichen wird. Hierdurch ist gewährleistet, daß der am stärksten gefährdete chemische Sensor sicher geschützt wird.

In einer Weiterbildung der Erfindung wird beim Nachregulieren des Verdünnungsverhältnisses ein neu einzustellender Wert des Verdünnungsverhältnisses aus einem ansteigenden Meßwertverlauf oder einem vorangegangenem Meßwert bestimmt und eingestellt. Darüber hinaus kann auch durch schrittweisen Vergleich mit den Detektionssignalen des chemischen Sensors ein vorgegebenes Meßwertoptimum bestimmt werden.

Schließlich kann ausgehend vom Detektionssignal des chemischen Sensors einer vorangegangenen Messung das voraussichtlich zu erwartende Detektionssignal der folgenden Messung bestimmt werden und hiernach das Verdünnungsverhältnis durch Gradientenbildung eingestellt werden. Hierdurch wird eine Vorhersage des nächsten Detektionssignales getroffen und dadurch eine schnellere Nachregulierung des Meßwertes erreicht. Dies ist insbesondere bei empfindlichen Detektionssystemen von Vorteil.

Es hat sich als zweckmäßig erwiesen, das Verdünnungsverhältnis über die Steuerung der Pumpenleistung einer oder mehrerer Pumpen, die das Probe-Verdünnungs- und/oder Gasgemisch fördern, zu regeln.

Die Regelung des Verdünnungsverhältnisses ist jedoch z.B. auch über ein oder mehrere Regelventile und/oder Massflow Controller oder über Magnetventile und Kapillaren gleicher oder unterschiedlicher öffnungsquerschnitte steuerbar, so daß dem Probegasstrom oder dem Verdünnungsgasstrom der jeweils andere zudosiert wird.

Erfindungsgemäß kann das Detektionssignal des chemischen Sensors oder der chemischen Sensoren in einem vorbestimmten Intervall des nominellen Meßbereiches sowohl durch schrittweise als auch stufenlose Veränderung des Verdünnungsverhältnisses gehalten werden. Eine schrittweise Veränderung des Verdünnungsverhältnisses kann beispielsweise in neun Verdünnungsschritten, beginnend bei einem Verdünnungsverhältnis von 1 : 256 in dualer Verminderungsreihenfolge bis zu einem Verdünnungsverhältnis von 1 : 1 vorgesehen werden.

Darüber hinaus ist es für den Einsatz bei chemischen Sensoren von Vorteil, einen linearen Anstieg bzw. Abfall des Verdünnungsverhältnisses vorzusehen. Typische Stoffkonzentrationsmeßbereiche für chemische und elektrochemische Sensoren liegen im Bereich zwischen 0 und 1 Vol.-% (0...10000ppm). Des weiteren gibt es auch Sensoren mit höheren Meßbereichen. Für diese ist die Verdünnung ebenfalls sinnvoll, um auch hohe Konzentrationen bis 100% messen zu können.

Das erfindungsgemäße Verfahren der dynamischen Verdünnung von gasförmigen Medien zur Einhaltung des Meßbereiches chemischer Sensoren nutzt somit die Verwendung eines Verdünnungsgasstromes bestimmter und vorteilhafterweise konstanter Zusammensetzung zur Vermischung mit dem Probegasstrom aus. Unter Verwendung des elektrischen Detektionssignales des chemischen Sensors wird festgestellt, ob der nominelle Meßbereich des chemischen Sensors eingehalten wird oder nicht. Liegt das Detektionssignal außerhalb dieses nominellen Meßbereiches bzw. außerhalb eines vorgegebenen Teilmeßbereiches innerhalb dieses nominellen Meßbereiches, so wird das Verdünnungsverhältnis von Probegasstrom und Verdünnungsgasstrom geändert. Die Ermittlung des Verdünnungsverhältnisses erfolgt dabei über eine Stoffkomponentenmessung im Probe-Verdünnungs- und/oder Gasgemischstrom.

Eine erfindungsgemäße Vorrichtung zur Durchführung der Messung eines Gasmediums mit mindestens einem chemischen Sensor sieht folgende Elemente vor:

a) Eine Einrichtung zum Entnehmen des Probegasstromes,

b) eine Einrichtung zum Bereitstellen eines Verdünnungsgasstromes,

c) eine Mischanordnung, um nach Maßgabe eines einstellbaren Verdünnungsverhältnisses den Verdünnungsgasstrom und den Probegasstrom zu einem Gasgemisch zu vermischen,

d) einen chemischen Sensor zum Erfassen einer Gasgemischkonzentration des Gasgemisches, und

e) eine Steuereinrichtung, vorteilhafterweise ein Mikroprozessor, um nach Maßgabe der Konzentration eines im Gasgemisch enthaltenen Stoffes, der mit mindestens einem weiteren Sensor erfaßt wird, das Verdünnungsverhältnis derart nachzuregulieren, daß die vom chemischen Sensor erfaßbare Gasgemischkonzentration im nominellen Meßbereich des chemischen Sensors gehalten wird.

Ein weiterer Sensor zum Erfassen des vorgegebenen Stoffes kann im Gasgemischstrom und/oder Probegasstrom angeordnet werden, abhängig davon, ob der vorgegebene Stoff im Probengasstrom vorhanden ist oder nicht.

Die Erfindung und deren Vorteile wird nachfolgend im Zusammenhang mit zwei Figuren beispielhaft näher erläutert. Es zeigen:

Figur 1     ein Prinzipschaltbild zur Durchführung des erfindungsgemäßen Verfahrens, und

Figur 2     eine detaillierte Darstellung des erfindungsgemäßen Verfahrens am Beispiel eines Detektionssystemes mit elektrochemischen Gassensoren und Sauerstoffensoren zur Ermittlung des Verdünnungsverhältnisses.

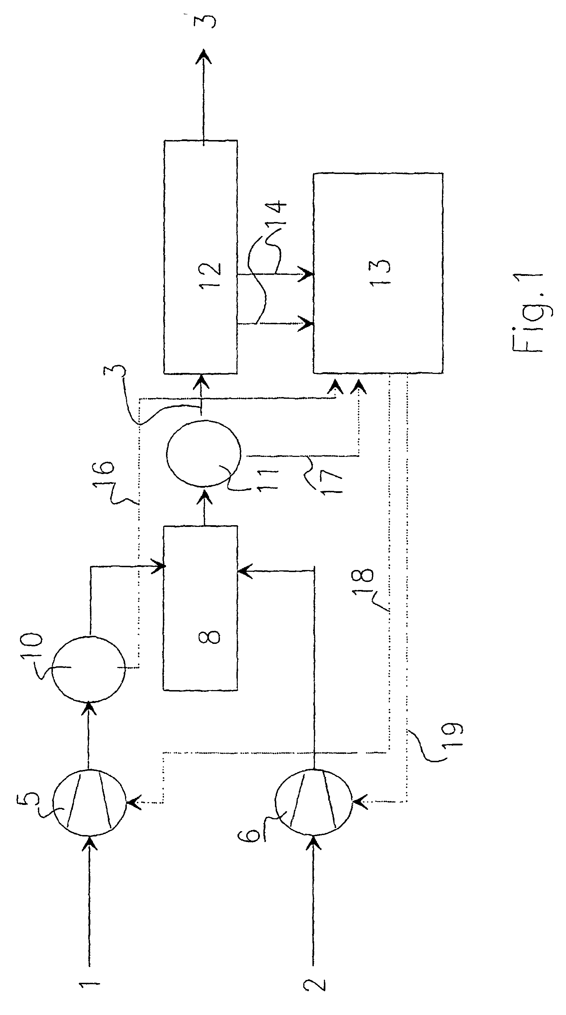

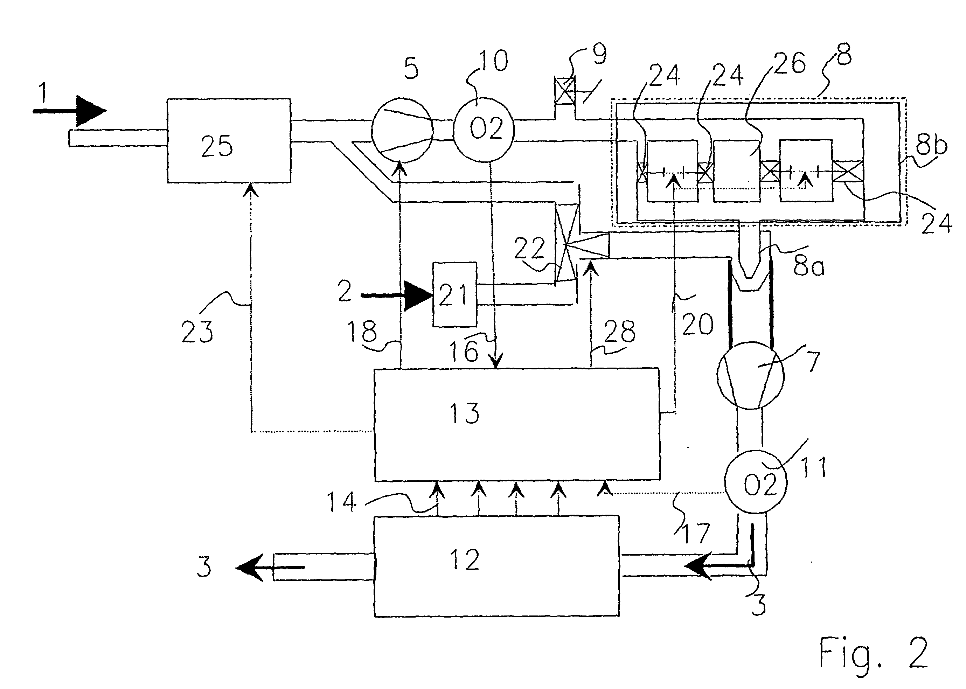

In den nachfolgenden Figuren 1 und 2 bezeichnen gleiche Bezugszeichen, sofern nicht anders angegeben, gleiche Teile mit gleicher Bedeutung.

In Figur 1 ist ein Probegasstrom mit dem Bezugszeichen 1 und ein Verdünnungsgasstrom mit dem Bezugszeichen 2 angegeben. Der Probegasstrom wird über eine Pumpe 5 einer Mischanordnung 8 zugeführt, während der Verdünnungsgasstrom 2 über eine Pumpe 6 der Mischanordnung 8 zugeführt wird. In der Zuführungsleitung von der Pumpe 5 zur Mischanordnung 8 ist ein Sensor 10 angeordnet. Der Ausgang der Mischanordnung 8 ist mit einer Detektionseinrichtung verbunden, in der mindestens ein chemischer Sensor 12 angeordnet ist. Diesem chemischen Sensor 12 wird ein Gasgemischstrom 3 zugeführt, der aus dem Probegasstrom 1 und Verdünnungsgasstrom 2 in einem vorgegebenen Verdünnungsverhältnis besteht. In der Zuleitung von Mischanordnung 8 zum chemischen Sensor 12 ist ein weiterer Sensor 11 angeordnet.

Zusätzlich ist eine Steuereinrichtung 13 vorgesehen, die vom chemischen Sensor 12 ein oder mehrere Detektionssignale 14 und von den weiteren Sensoren 10, 11 weitere Sensorsignale 16, 17 erhält. Abhängig von diesen Detektionssignalen 14 und Sensorsignalen 16, 17 steuert die Steuereinrichtung 13 über Einstellsignale 18, 19 die Pumpleistung der Pumpen 5 und 6.

Zur gezielten Einstellung der Verdünnung des Probegasstromes 1 und damit zum Einhalten des nominellen Meß- bzw. optimalen Teilmeßbereichs des chemischen Sensors 12 dient die Steuereinrichtung 13, die die Pumpleistungen der Pumpen 5 und 6 steuert. Zur Erzielung eines korrekten Meßwertes ist es notwendig, das Verdünnungsverhältnis exakt zu kennen, um das Detektionssignal nach Maßgabe dieses Verdünnungsverhältnisses zu korrigieren, um schließlich den realen Konzentrationswert des Gasmediums festzustellen.

Zur Bestimmung des Verdünnungsverhältnisses dient erfindungsgemäß eine Stoffkonzentrationsmessung.

Im Beispiel von Figur 1 ist angenommen, daß die Stoffkomponente im Verdünnungsgasstrom 2 in einem bekannten Verhältnis vorliegt und im Probegasstrom 1 ebenfalls vorhanden ist. Erfindungsgemäß braucht deshalb lediglich in der Zuleitung von der Pumpe 5 zur Mischanordnung 8 ein Sensor 10 und in der Zuleitung von der Mischanordnung 8 zum chemischen Sensor 12 ein weiterer Sensor 11 vorgesehen zu werden, während ein solcher Sensor in der Zuleitung des Verdünnungsgasstromes 2 zur Mischanordnung 8 entfallen kann.

Ist X die Stoffgröße, über die das Verdünnungsverhältnis bestimmt werden soll, so ergibt sich der Verdünnungsfaktor nach folgender Formel:

$$\text{Verdünnungsfaktor} = \left( \frac{X2 - X1}{X2 - X3} \right) ,$$

wobei die Ziffern 1, 2 und 3 wieder für den Probegasstrom, den Verdünnungsgasstrom und den Gasgemischstrom stehen. Wird Luft als Verdünnungsmedium eingesetzt, so wäre für X2 etwa 21 Vol.-% einzusetzen. Der reale Konzentrationswert ergibt sich folglich aus dem Produkt aus Sensormeßwert des chemischen Sensors 12 und Verdünnungsfaktor.

Im Ausführungsbeispiel von Figur 1 ist angenommen, daß die Stoffkomponente im Verdünnungsgasstrom in bekannter und konstanter Größe vorliegt. Als vorgegebene Stoffkomponente könnte beispielsweise Sauerstoff in Betracht kommen. Ist der Bestandteil Sauerstoff im Probegasstrom 1 nicht vorhanden, so könnte sogar der weitere Sensor 10 in der in Figur 1 dargestellten Anordnung entfallen. Das Verdünnungsverhältnis ergibt sich dann ausschließlich aus dem vom weiteren Sensor 11 erfaßten Sauerstoffgehaltswert.

Das erfindungsgemäße Verfahren zur dynamischen Regelung der Verdünnung des zu detektierenden Probegasstromes 1 bei Einhaltung des Arbeitsbereiches des chemischen Sensors 12 kann beispielsweise in folgenden Schritten durchgeführt werden:

1. Vorgabe einer maximal möglichen Konzentration des chemischen Sensors 12 und daraus ableitend die Voreinstellung des maximalen Verdünnungsverhältnisses.

2. Berechnung des neuen Verdünnungsverhältnisses in der Steuereinrichtung 13 aus dem Meßwert des chemischen Sensors 12 oder einem durch die Trägheit des Detektionssystemes mit dem chemischen Sensor 12 bestimmten Anstiegsverhalten des Meßwertes und dynamische Nachstellung über die Steuereinrichtung 13 in Verbindung mit Stellapparaturen, wie z. B. die Pumpen 5, 6, Magnet- und/oder Regelventile, Massflow-Controller, etc.

3. Bestimmung des aktuell eingestellten Mischungs- bzw. Verdünnungsverhältnisses durch eine Stoffkonzentrationsmessung unter Verrechnung dieses Wertes mit der aktuellen Sensorkonzentration des chemischen Sensors 12 zur Bestimmung der real vorhandenen Stoffkonzentration im Probegasstrom 1.

4. Dynamische Nachregulierung des Verdünnungsverhältnisses über die Sensorkonzentration bei schwankender Konzentrationszusammensetzung des Probegasstromes 1 derart, daß die Sensorkonzentration des chemischen Sensors 12 in einem Teilmeßbereich um einen definiert eingestellten Wert, insbesondere durch schrittweise, intervallmäßige oder stufenlose Veränderung des Verdünnungsverhältnisses gehalten wird.

5. Ist ein Mehrkomponentenmeßsystem, also ein Detektionssystem mit einer Vielzahl von unterschiedlichen chemischen Sensoren 12 vorgesehen, kann durch schritt- oder wahlweises Entfernen des jeweils höchstbelasteten chemischen Sensors 12 aus dem Meßgasweg des Gasgemischstromes 3 die Messung der verbliebenen Komponenten im entsprechenden optimalen Teilmeßbereich durch wiederholte dynamische Nachregulierung des Verdünnungsverhältnisses erfolgen.

Durch ein derartiges Vorgehen ist es möglich, Konzentrationen mit einem chemischen Sensor 12 zu messen, die außerhalb des nominellen Meßbereichs des chemischen Sensors 12 liegen und bei Veränderung im Probegasstrom 1 dynamisch die Verdünnung so nachzuregulieren, daß die Meßwerte des chemischen Sensors 12 weiterhin im zulässigen Meßbereich verbleiben und eine Gefährdung des chemischen Sensors 12 vermieden wird.

In Figur 2 ist ein detaillierteres Schaltbild am Beispiel eines Detektorsystemes mit elektrochemischen Gassensoren gezeigt. Der Probegasstrom 1 wird über eine Aufbereitungseinrichtung 25, in der der Probegasstrom 1 dem zu messenden Gasstrom entnommen und in geeigneter Weise aufbereitet wird, durch die Pumpe 5 in die Mischanordnung 8 gefördert. Der Sensor 10 in der Zuleitung des Probegasstromes 1 zur Mischanordnung 8 dient zum Erfassen des Sauerstoffgehaltes, also des $O_2$-Gehaltes. Hierdurch wird der Probegasstrom 1 auf seinen Inhalt an Sauerstoff analysiert. Durch ein Überdruckablaßventil 9 in der Zuleitung zur Mischanordnung 8 wird in der Mischanordnung 8 druckseitig ein vorteilhafterweise konstanter Überdruck realisiert.

Der Verdünnungsgasstrom 2, im vorliegenden Fall wieder ein Luftstrom, wird über ein Filter 21, hier ein Luftfilter, und ein Ventil 22, das als Dreiwegeventil ausgebildet ist, der Mischanordnung 8 zugeführt.

Die Mischanordnung 8 weist eine Dosiereinrichtung 8b und eine Mischkammer 8a auf. Die Dosiereinrichtung 8b ist mit Kapillaren 24, die vorzugsweise unterschiedliche Durchmesser aufweisen und Ventilen 26 ausgestattet. Die Steuereinrichtung 13 steuert die mit den Kapillaren 24 in Verbindung stehenden Ventile 26 derart, daß eine vorgegebene Menge des Probegasstromes 1 in die Mischkammer 8a gelangt. In dieser Mischkammer 8a wird der vorgegebenen Menge an Probegasstrom 1 der Verdünnungsgasstrom 2 in Form des Luftgasstromes hinzugefügt. Der Luftgasstrom wird über das Ventil 22 geführt.

Die Ventile 26 der Dosiereinrichtung 8b sind vorzugsweise Magnetventile, durch deren Öffnungen unterschiedliche Verdünnungsverhältnisse, die durch Abstimmung zwischen den Kapillaröffnungsquerschnitten und der Pumpleistung der nach der Mischkammer 8a angeordneten Pumpe 7 grob vorgegeben sind. Die Pumpe 7 saugt über den Filter 21 und das Ventil 22 einen Luftstrom an, dem in der Mischkammer 8a der Probegasstrom 1 hinzudosiert wird.

Ausgangsseitig ist die Pumpe 7 an ein Detektionssystem mit mindestens einem chemischen Sensor 12 in Form eines elektrochemischen Gassensors angeschlossen. Hierdurch wird dem chemischen Sensor 12 der Gasgemischstrom 3, der sich aus dem Probegasstrom 1 und dem Luftgasstrom 2 in einem vorgegebenen Verdünnungsverhältnis zusammensetzt, zugeführt. Im Gasgemischstrom 3 ist zusätzlich ein weiterer Sensor 11, hier wieder ein $O_2$-Sensor, zum Erfassen der Sauerstoffkonzentration im Gasgemischstrom 3 angeordnet. Der weitere Sensor 11 ermöglicht zusammen mit dem Meßsignal des Sensors 10, der ebenfalls ein $O_2$-Sensor ist, die Bestimmung des Verdünnungsverhältnisses gemäß der oben angegebenen Formel.

Die Berechnung des Verdünnungsverhältnisses erfolgt in der Steuereinrichtung 13. Der im Detektionssystem mindestens eine angeordnete chemische Sensor 12 analysiert den Gasgemischstrom 3 und überträgt das Meßergebnis in Form eines oder mehrerer Detektionssignale 14 an die Steuereinrichtung 13. Als weitere Eingangssignale empfängt die Steuereinrichtung 13 die Sensorsignale 16 und 17 der weiteren Sensoren 10 und 11. über Einstellsignale 18, 20 und Steuersignale 23 und 28 steuert die Steuereinrichtung 13 die Pumpleistung der Pumpe 5, die Ventile innerhalb der Dosiereinrichtung 8b zur Nachregelung des Verdünnungsverhältnisses, die Aufbereitungseinrichtung 25 sowie das Ventil 22.

Sind im Detektionssystem mehrere chemische Sensoren 12 zur Detektion mehrerer Stoffgrößen des Gasgemischstromes 3 vorhanden, so erfolgt eine Regelung des Verdünnungsverhältnisses vorzugsweise nach Maßgabe eines Detektionssignales 14 desjenigen chemischen Sensors 12, von dessen nominellen Meßbereich zunächst die größte Abweichung erwartet wird. Hierdurch ist gewährleistet, daß dieser am stärksten gefährdete chemische Sensor 12 geschützt wird.

Zu Beginn des Meßvorganges wird das Verdünnungsverhältnis so groß eingestellt, daß der oder die chemischen Sensoren 12 im Detektionssystem sicher in ihrem nominellen Meßbereich arbeiten. Es hat sich als günstig erwiesen, hier zunächst die maximal mögliche Verdünnung einzustellen.

Zur Erzielung einer schnellen Nachregulierung des Verdünnungsverhältnisses, das stufenweise oder stufenlos erfolgen kann, wird ausgehend vom Detektionssignal 14 des chemischen Sensors 12 einer vorangegangenen Messung das voraussichtlich zu erwartende Detektionssignal 14 der folgenden Messung in der Steuereinrichtung 13 vorab bestimmt und davon ausgehend das Verdünnungsverhältnis durch Gradientenbildung eingestellt.

## FIGURENLEGENDE

1    Probegasstrom
2    Verdünnungsgasstrom
3    Gasgemisch
5    Pumpe
6    Pumpe
7    Pumpe
8    Mischanordnung
9    Überdruckablaßventil

10    Sensor

| 11 | Sensor |
|---|---|
| 12 | chemischer Sensor |
| 13 | Steuereinrichtung |
| 14 | Detektionssignal |
| 16 | Sensorsignal |
| 17 | Sensorsignal |
| 18 | Einstellsignal |
| 19 | Einstellsignal |

| 20 | Einstellsignal |
|---|---|
| 21 | Filter |
| 22 | Ventil |
| 23 | Steuersignal |
| 24 | Kapillare |
| 25 | Aufbereitungseinrichtung |
| 26 | Ventil |
| 28 | Steuersignal |

$O_2$ vorgegebene Stoffkomponente

| 8a | Mischkammer |
|---|---|
| 8b | Dosiereinrichtung |

**Patentansprüche**

**1.** Verfahren zum Messen einer ersten Messgrösse in einem Gasmedium mit einem chemischen Sensor und folgenden Merkmalen:

a) Entnehmen eines Probegasstromes (1) aus dem Gasmedium,

b) Erzeugen eines Gasgemisches (3) durch Vermischen des Probegasstromes (1) mit einem Verdünnungsgasstrom (2) in einem einstellbaren Verdünnungsverhältnis, wobei im Verdünnungsgasstrom eine vorgegebene Stoffkomponente ($O_2$) in einem bekannten Verhältnis vorliegt,

c) Bestimmung des Verdünnungsverhältnisses durch Messen einer zweiten Messgrösse nach dem Mischvorgang, wobei die zweite Messgröße ein Maß für eine Stoffkonzentra tion der vorgegebenen Stoffkomponente (O2) ist,

d) Zuführen des Gasgemisches (3) an einen chemischen Sensor (12) mit einem nominellen Meßbereich zum Erzeugen eines Detektionssignales (14)

e) Nachregulieren des Verdünnungsverhältnisses durch Auswertung des Detektionssignales (14) derart, dass die vom chemischen Sensor (12) erfaßbare Gasgemischkonzentration im nominellen Messbereich des chemischen Sensors (12) gehalten wird, und

f) Ermitteln eines Messwertes für die erste Messgrösse durch Auswertung des Detektionssignales (14) unter Berücksichtigung des Verdünnungsverhältnisses.

**2.** Verfahren nach Anspruch 1, **dadurch gekennzeichnet, daß** die vorgegebene Stoffkomponente ($O_2$) im Verdünnungsgasstrom (2) in bekannter und konstanter Größe vorliegt und eine Stoffkonzentrationsmessung dieser Stoffkomponente ($O_2$) im Probengasstrom (1) und Gasgemischstrom (3) erfolgt.

**3.** Verfahren nach Anspruch 1, **dadurch gekennzeichnet, daß** die vorgegebene Stoffkomponente ($O_2$) im Probengasstrom (1) nicht und im Verdünnungsgasstrom (2) in bekannter und konstanter Größe vorliegt und eine Stoffkonzentrationsmessung dieser Stoffkomponente ($O_2$) ausschließlich im Gasgemischstrom (3) erfolgt.

**4.** Verfahren nach einem der Ansprüche 1 bis 3, **dadurch gekennzeichnet, daß** die vorgegebene Stoffkomponente

($O_2$) Sauerstoff ist.

**5.** Verfahren nach einem der Ansprüche 1 bis 4, **dadurch gekennzeichnet, daß** als Verdünnnungsgasstrom (2) ein Luftstrom verwendet wird.

**6.** Verfahren nach einem der Ansprüche 1 bis 5, **dadurch gekennzeichnet, daß** zu Beginn des Verfahrens das Verdünnungsverhältnis so groß eingestellt wird, daß der chemische Sensor (12) sicher in seinem nominellen Meßbereich arbeitet.

**7.** Verfahren nach einem der Ansprüche 1 bis 6, **dadurch gekennzeichnet, daß** mehrere chemische Sensoren (12) zur Detektion mehrerer Stoffgrößen des Gasmediums vorgesehen sind und eine Regelung des Verdünnungsverhältnisses nach Maßgabe eines Detektionssignales (14) desjenigen chemischen Sensors (12) erfolgt, von dessen nominellem Meßbereich zu Beginn des Verfahrens am weitesten abgewichen wird.

**8.** Verfahren nach einem der Ansprüche 1 bis 7, **dadurch gekennzeichnet, daß** beim Nachregulieren des Verdünnungsverhältnisses ein neu einzustellender Wert des Verdünnungsverhältnisses aus einem ansteigenden Meßwertverlauf oder einem vorangegangenen Meßwert bestimmt und eingestellt wird.

**9.** Verfahren nach einem der Ansprüche 1 bis 7, **dadurch gekennzeichnet, daß** ausgehend vom Detektionssignal (14) des chemischen Sensors (12) einer vorangegangenen Messung das voraussichtlich zu erwartende Detektionssignal (14) der folgenden Messung bestimmt und davon ausgehend das Verdünnungsverhältnis durch Gradientenbildung eingestellt wird.

**10.** Verfahren nach einem der Ansprüche 1 bis 9, **dadurch gekennzeichnet, daß** das Detektionssignal (14) des chemischen Sensors (12) in einem vorbestimmten Intervall des nominellen Meßbereiches durch schrittweise oder stufenlose Veränderung des Verdünnungsverhältnisses gehalten wird.

**11.** Vorrichtung zur Durchführung des Verfahrens nach einem der Ansprüche 1 bis 10, **gekennzeichnet durch** die Merkmale:

a) Eine Einrichtung (25) zum Entnehmen des Probegasstromes (1),

b) eine Einrichtung (21) zum Bereitstellen eines Verdünnungsgasstromes (2),

c) eine Mischanordnung (8), um nach Maßgabe eines einstellbaren Verdünnungsverhältnisses den Verdünnungsgasstrom (2) und Probegasstrom (1) zu einem Gasgemisch (3) zu vermischen,

d) einen chemischen Sensor (12) zum Erfassen einer Gasgemischkonzentration des Gasgemisches (3) und

e) eine Steuereinrichtung (13), um nach Maßgabe einer mittels mindestens eines weiteren Sensors (10, 11) erfaßbaren Konzentration einer im Gasgemisch enthaltenen Stoffkomponente ($O_2$) das Verdünnungsverhältnis derart nachzuregulieren, daß die vom chemischen Sensor (12) erfaßbare Gasgemischkonzentration im nominellen Meßbereich des chemischen Sensors (12) gehalten wird.

**12.** Vorrichtung nach Anspruch 11, **dadurch gekennzeichnet, daß** einer der weiteren Sensoren (11) zum Erfassen der vorgegebenen Stoffkomponente ($O_2$) im Gasgemischstrom (3) angeordnet ist.

**13.** Vorrichtung nach Anspruch 11 oder 12, **dadurch gekennzeichnet, daß** ein Sensor (10) zum Erfassen der vorgegebenen Stoffkomponente ($O_2$) im Probegasstrom (1) angeordnet ist.

**14.** Vorrichtung nach Anspruch 12 oder 13, **dadurch gekennzeichnet, daß** die weiteren Sensoren (10, 11) zum Erfassen der vorgegebenen Stoffkomponente ($O_2$) Sauerstoffsensoren sind.

**15.** Vorrichtung nach einem der Ansprüche 11 bis 14, **dadurch gekennzeichnet, daß** als Verdünnungsgasstrom (2) ein Luftstrom vorgesehen ist.

**16.** Vorrichtung nach einem der Ansprüche 11 bis 15, **dadurch gekennzeichnet, daß** die Mischanordnung (8) eine vorgegebene Anzahl von Kapillaren (24) mit unterschiedlichem Durchmesser und jeweils ein zugeordnetes Ventil

**EP 0 670 490 B1**

(26) aufweist.

17. Vorrichtung nach einem der Ansprüche 11 bis 16, **dadurch gekennzeichnet, daß** in einer Zuleitung für den Probegasstrom (1) zur Mischanordnung (8) ein Überdruck einstellbar ist.

18. Vorrichtung nach Anspruch 17, **dadurch gekennzeichnet, daß** in der den Probegasstrom (1) führenden Zuleitung zur Mischanordnung (8) ein Überdruckablaßventil (9) angeordnet ist.


**Claims**

1. Method for the measurement of a first measured quantity in a gas medium with a chemical sensor and the following features:

   a) removal of a test gas stream (1) from the gas medium,
   b) creation of a gas mixture (3) by mixing the test gas stream (1) with a diluting gas stream (2) in an adjustable dilution ratio, there being a defined substance content ($O_2$) present in a known ratio in the diluting gas stream,
   c) determination of the dilution ratio by measuring a second measured quantity after the mixing process, the second measured quantity being a measure of a substance concentration of the defined substance component ($O2$),
   d) feeding of the gas mixture (3) to a chemical sensor (12) with a nominal measuring range to produce a detection signal (14)
   e) readjustment of the dilution ratio by evaluation of the detection signal (14) in such a way that the gas mixture concentration, which can be detected by the chemical sensor (12), is maintained within the nominal measuring range of the chemical sensor (12), and
   f) determination of a measured value for the first measured quantity by evaluation of the detection signal (14) taking into account the dilution ratio.

2. Method according to Claim 1, **characterised in that** the defined substance component ($O_2$) is present in the diluting gas stream (2) in known and constant magnitude and a measurement of the substance concentration of this substance component ($O_2$) takes place in the test gas stream (1) and gas mixture stream (3).

3. Method according to Claim 1, **characterised in that** the defined substance component ($O_2$) is not present in the test gas stream (1) and is present in the diluting gas stream (2) in known and constant magnitude and a measurement of the substance concentration of this substance component ($O_2$) takes place exclusively in the gas mixture stream (3).

4. Method according to one of Claims 1 to 3, **characterised in that** the defined substance component ($O_2$) is oxygen.

5. Method according to one of Claims 1 to 4, **characterised in that** a stream of air is used as the diluting gas stream (2).

6. Method according to one of Claims 1 to 5, **characterised in that**, at the start of the method, the dilution ratio is set sufficiently high that the chemical sensor (12) works reliably in its nominal measuring range.

7. Method according to one of Claims 1 to 6, **characterised in that** several chemical sensors (12) for the detection of several substance quantities of the gas medium are provided and a control of the dilution ratio takes place according to a detection signal (14) from that chemical sensor (12) for which the deviation from the nominal measuring range at the start of the method is the highest.

8. Method according to one of Claims 1 to 7, **characterised in that**, when readjusting the dilution ratio, a value of the dilution ratio to be newly set is determined and set from an increasing measured value characteristic or from a previous measured value.

9. Method according to one of Claims 1 to 7, **characterised in that**, starting from the detection signal (14) of the chemical sensor (12) of a previous measurement, the detection signal (14) presumably to be expected from the following measurement is determined and from this the dilution ratio is set by forming the gradient.

9

**EP 0 670 490 B1**

10. Method according to one of Claims 1 to 9, **characterised in that** the detection signal (14) of the chemical sensor (12) is maintained within a predetermined interval of the nominal measuring range by stepwise or stepless changing of the dilution ratio.

11. Apparatus for carrying out the method according to one of Claims 1 to 10, **characterised by** the features:

   a) a device (25) for the removal of the test gas stream (1),
   b) a device (21) for the provision of a diluting gas stream (2),
   c) a mixing arrangement (8) to mix the diluting gas stream (2) and test gas stream (1) to form a gas mixture (3) according to an adjustable dilution ratio,
   d) a chemical sensor (12) for the detection of a gas mixture concentration of the gas mixture (3) and
   e) a control unit (13) to readjust the dilution ratio according to a concentration of a substance component ($O_2$), contained in the gas mixture, which can be detected by means of at least one further sensor (10, 11), in such a way that the gas mixture concentration, which can be detected by the chemical-sensor (12), is maintained within the nominal measuring range of the chemical sensor (12).

12. Apparatus according to Claim 11, **characterised in that** one of the further sensors (11) for detecting the defined substance component ($O_2$) is arranged in the gas mixture stream (3).

13. Apparatus according to Claim 11 or 12, **characterised in that** one sensor (10) for detecting the defined substance component ($O_2$) is arranged in the test gas stream (1).

14. Apparatus according to Claim 12 or 13, **characterised in that** the further sensors (10, 11) for detecting the defined substance component ($O_2$) are oxygen sensors.

15. Apparatus according to one of Claims 11 to 14, **characterised in that** a stream of air is provided as a diluting gas stream (2).

16. Apparatus according to one of Claims 11 to 15, **characterised in that** the mixing arrangement (8) has a defined number of capillaries (24) with different diameter and each with an associated valve (26).

17. Apparatus according to one of Claims 11 to 16, **characterised in that** it is possible to set an overpressure in a feed line for the test gas stream (1) to the mixing arrangement (8).

18. Apparatus according to Claim 17, **characterised in that** an overpressure relief valve (9) is arranged in the feed line carrying the test gas stream (1) to the mixing arrangement (8).

**Revendications**

1. Procédé de mesure d'une première grandeur dans un milieu gazeux avec un capteur chimique ayant les caractéristiques suivantes :

   a) on prélève une veine de gaz échantillon (1) dans le milieu gazeux,
   b) on génère un mélange de gaz (3) en mélangeant la veine de gaz échantillon (1) à une veine de gaz de dilution (2), selon un rapport de dilution réglable, avec dans la veine de gaz de dilution, une composante prédéterminée de matière ($O_2$) selon un rapport connu,
   c) on détermine le rapport de dilution en mesurant une seconde grandeur de mesure après l'opération de mélange, cette seconde grandeur de mesure étant une mesure d'une concentration du composant prédéterminé ($O_2$),
   d) on fournit le mélange gazeux (3) à un capteur chimique (12) ayant une plage de mesure nominale pour générer un signal de détection (14),
   e) on asservit le rapport de dilution par l'exploitation du signal de détection (14) de façon que la concentration de mélange gazeux saisie par le capteur chimique (12) soit maintenue dans une plage de mesure nominale du capteur chimique (12), et
   f) on détermine une valeur de mesure de la première grandeur de mesure par l'exploitation du signal de détection (14) en tenant compte du rapport de dilution.

10

**2.** Procédé selon la revendication 1,
**caractérisé en ce que**
la composante de matière prédéterminée ($O_2$) est présente dans la veine de gaz de dilution (2) selon une grandeur connue et constante et on effectue une mesure de concentration de matière de ce composant ($O_2$) dans la veine de gaz échantillon (1) et dans la veine de gaz de mélange (3).

**3.** Procédé selon la revendication 1,
**caractérisé en ce que**
la composante de matière prédéterminée ($O_2$) n'existe pas dans la veine de gaz échantillon (1) et existe dans la veine de gaz de dilution (2) selon une grandeur connue et constante, et on effectue une mesure de concentration de matière de ce composant ($O_2$) exclusivement dans la veine de gaz de mélange (3).

**4.** Procédé selon l'une des revendications 1 à 3,
**caractérisé en ce que**
la composante de matière prédéterminée ($O_2$) est de l'oxygène.

**5.** Procédé selon l'une des revendications 1 à 4,
**caractérisé en ce que**
la veine de gaz de dilution (2) est une veine d'air.

**6.** Procédé selon l'une des revendications 1 à 5,
**caractérisé en ce qu'**
au début du procédé, on règle le rapport de dilution à un niveau élevé pour que le capteur chimique (2) travaille de façon garantie dans sa plage de mesure nominale.

**7.** Procédé selon l'une des revendications 1 à 6,
**caractérisé en ce qu'**
on utilise plusieurs capteurs chimiques (12) pour détecter plusieurs grandeurs de matière du milieu gazeux et on régule le rapport de dilution selon l'indication d'un signal de détection (14) de celui des capteurs chimiques (12) qui est le plus éloigné de sa plage de mesure nominale au début du procédé.

**8.** Procédé selon l'une des revendications 1 à 7,
**caractérisé en ce qu'**
en asservissant le rapport de dilution, on détermine et on règle une nouvelle valeur du rapport de dilution à partir d'une courbe de valeur de mesure croissante ou d'une valeur de mesure antérieure.

**9.** Procédé selon l'une des revcndications 1 à 7,
**caractérisé en ce que** partant du signal de détection (14) du capteur chimique (12) d'une mesure antérieure, on détermine le signal de détection (14) prévisible de la mesure suivante et, à partir de là, on règle le rapport de dilution en formant des gradients.

**10.** Procédé selon l'une des revendications 1 à 9,
**caractérisé en ce que**
le signal de détection (14) du capteur chimique (12) est maintenu dans un intervalle prédéterminé de la plage de mesure nominale par une variation pas à pas ou continue du rapport de dilution.

**11.** Dispositif pour la mise en oeuvre du procédé selon l'une des revendications 1 à 10,
**caractérisé en ce qu'**
il comprend :

a) une installation (25) pour prélever la veine de gaz échantillon (1),
b) une installation (21) pour fournir une veine de gaz de dilution (2),
c) un dispositif mélangeur (8) pour que, selon l'indication, il mélange, selon un rapport de dilution réglable, la veine de gaz de dilution (2) et la veine de gaz échantillon (1) pour former un mélange gazeux (3),
d) un capteur chimique (12) pour détecter une concentration dans le mélange gazeux (3) et
e) une installation de commande (13) pour qu'en fonction d'une concentration détectée à l'aide d'au moins un autre capteur (10, 11) d'une composante de matière ($O_2$) contenue dans le mélange gazeux, on asservit le rapport de dilution de façon que la concentration de mélange gazeux saisi par le capteur chimique (12) reste dans la plage de mesure nominale du capteur chimique (12).

12. Dispositif selon la revendication 11,
    **caractérisé par**
    un autre capteur (11) pour détecter la composante de matière prédéterminée ($O_2$) dans la veine de mélange gazeux (3).

13. Dispositif selon l'une des revendications 11 ou 12,
    **caractérisé par**
    un capteur (10) pour détecter la composante de matière prédéterminée ($O_2$) dans la veine de gaz échantillon (1).

14. Dispositif selon l'une des revendications 12 ou 13,
    **caractérisé en ce que**
    les autres capteurs (10, 11) sont prévus pour détecter la composante de matière prédéterminée ($O_2$) sont des capteurs d'oxygène.

15. Dispositif selon l'une des revendications 11 à 14,
    **caractérisé en ce que**
    la veine de gaz de dilution (2) est une veine d'air.

16. Dispositif selon l'une des revendications 11 à 15,
    **caractérisé en ce que**
    le dispositif mélangeur (8) comporte un certain nombre de tubes capillaires (24) de diamètres différents et, chaque fois, une vanne (26) associée.

17. Dispositif selon l'une des revendications 11 à 16,
    **caractérisé en ce qu'**
    on règle une surpression dans une conduite d'alimentation de la veine de gaz échantillon (1) vers le mélangeur (8).

18. Dispositif selon la revendication 17,
    **caractérisé par**
    une soupape de surpression (9) dans la conduite fournissant la veine de gaz échantillon (1) au mélangeur (8).

Fig.1

EP 0 670 490 B1

Fig. 2

EP 0 670 490 B1